(19) Europäisches Patentamt
European Patent Office
Office européen des brevets

(11) EP 3 833 410 B1

(12) EUROPÄISCHE PATENTSCHRIFT

(45) Veröffentlichungstag und Bekanntmachung des Hinweises auf die Patenterteilung:
08.10.2025 Patentblatt 2025/41

(21) Anmeldenummer: 19752180.0

(22) Anmeldetag: 07.08.2019

(51) Internationale Patentklassifikation (IPC):
A61M 60/13 (2021.01)     A61M 60/183 (2021.01)
A61M 60/221 (2021.01)    A61M 60/804 (2021.01)
A61M 60/82 (2021.01)     A61M 60/824 (2021.01)
A61M 60/825 (2021.01)

(52) Gemeinsame Patentklassifikation (CPC):
A61M 60/824; A61M 60/13; A61M 60/183;
A61M 60/221; A61M 60/804; A61M 60/82;
A61M 60/825

(86) Internationale Anmeldenummer:
PCT/EP2019/071233

(87) Internationale Veröffentlichungsnummer:
WO 2020/030700 (13.02.2020 Gazette 2020/07)

(54) **LAGERVORRICHTUNG FÜR EIN HERZUNTERSTÜTZUNGSSYSTEM UND VERFAHREN ZUM SPÜLEN EINES ZWISCHENRAUMS IN EINER LAGERVORRICHTUNG FÜR EIN HERZUNTERSTÜTZUNGSSYSTEM**

BEARING DEVICE FOR A HEART SUPPORT SYSTEM, AND METHOD FOR RINSING A SPACE IN A BEARING DEVICE FOR A HEART SUPPORT SYSTEM

DISPOSITIF DE SUPPORT DESTINÉ À UN DISPOSITIF D'ASSISTANCE VENTRICULAIRE ET PROCÉDÉ DESTINÉ À RINCER UN ESPACE DANS UN DISPOSITIF DE SUPPORT POUR UN DISPOSITIF D'ASSISTANCE VENTRICULAIRE

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR

(30) Priorität: 07.08.2018 DE 102018213150

(43) Veröffentlichungstag der Anmeldung:
16.06.2021 Patentblatt 2021/24

(73) Patentinhaber: Kardion GmbH
70376 Stuttgart (DE)

(72) Erfinder:
• STOTZ, Ingo
71254 Ditzingen (DE)
• EIBERGER, Fabian
88690 Uhldingen- Mühlhofen (DE)

(74) Vertreter: Gauss, Nikolai et al
Pfiz/Gauss Patentanwälte PartmbB
Tübingerstraße 26
70178 Stuttgart (DE)

(56) Entgegenhaltungen:
WO-A1-2016/146661     WO-A1-2017/021465
WO-A2-2008/017289

## Beschreibung

Die Erfindung betrifft eine Lagervorrichtung für ein Herzunterstützungssystem mit einer Standeinheit, mit einem Impeller und mit einem zwischen dem Impeller und der Standeinheit ausgebildeten Zwischenraum zum Führen eines Spülfluidstroms aus einem Fluid, wobei die Standeinheit einen in den Impeller ragenden Teilabschnitt aufweist und ausgeformt ist, den Impeller um eine Drehachse drehbar zu lagern, wobei der Impeller ausgebildet ist, sich bei einem Betrieb des Herzunterstützungssystems zum Fördern eines Pumpenfluidstroms aus dem Fluid in eine Flussrichtung um eine mit der Drehachse fluchtende Längsachse zu drehen, und wobei der Impeller wenigstens einen Spülausgang für das Ausleiten des Spülfluidstroms aus dem Zwischenraum aufweist. Der wenigstens eine Spülausgang weist eine Austrittsöffnung für das Austreten des Spülfluidstroms auf, die einen Öffnungsquerschnitt hat, bei dem an wenigstens einer Stelle ein Öffnungsquerschnittsnormalenvektor eine der Drehachse abgewandte und zu der Drehachse radiale Richtungskomponente aufweist, wobei sich der Impeller in einem Gehäuse mit einem Gehäuseabschnitt befindet, an den ein Zulaufschlauch für das Zuführen des Fluids angeschlossen ist.

Darüber hinaus betrifft die Erfindung ein Herzunterstützungssystem mit einer Lagervorrichtung sowie ein Verfahren zum Spülen eines Zwischenraums für das Führen eines Spülfluidstroms mit einem Fluid in einer Lagervorrichtung für ein Herzunterstützungssystem sowie ein Verfahren zum Herstellen einer Lagervorrichtung für ein Herzunterstützungssystem.

Zur Herz-Kreislauf-Unterstützung herzinsuffizienter Patienten werden insbesondere Systeme eingesetzt, die einen Teil oder die komplette Pumpfunktion des Herzens übernehmen. Diese Systeme, auch Herzunterstützungssysteme oder kurz VAD (ventricular assist device) genannt, können in temporäre Systeme zur kurzeitigen Herzunterstützung und dauerhafte Systeme zum langzeitigen Verbleib am oder im Patienten untergliedert werden. Bestandteil eines solchen Systems ist in der Regel eine Blutpumpe, typischerweise eine Kreiselpumpe (Turbopumpe), die durch einen integrierten Elektromotor angetrieben wird und mittels eines Laufrades den geforderten Blutfluss erzeugt. Die Pumpe kann hierbei an unterschiedlichen Stellen implantiert werden. Beispielsweise kann die Pumpe durch eine invasive Operation per Sternotomie von außen an das Herz angenäht werden oder minimalinvasiv durch einen Katheter in der Aorta oder im Ventrikel abgesetzt werden. Im letzteren Fall ist der maximal zulässige Außendurchmesser der Pumpe in der Regel auf 10 mm limitiert, weshalb der Einsatz einer Pumpe axialer Bauart mit einem axial angeströmten Laufrad anzustreben ist. Dabei wird das zu fördernde Blut durch am Umfang eines zylindrischen Pumpengehäuses angebrachte Austrittsöffnungen ausgestoßen, um wieder der Aorta zugeführt zu werden.

Aus der EP 3 127 562 A1 ist eine Blutpumpe für ein Herzunterstützungssystem bekannt, die ein Pumpengehäuse mit einem Impeller aufweist, der in dem Pumpengehäuse in einem Gleitlager drehbar gelagert ist, das stationäre Lagerflächen hat, an denen an den Schaufeln des Impellers ausgebildete Lagerflächen anliegen. Die komplexe Struktur der Schaufeln des Impellers, an dem die Lagerflächen ausgebildet sind, bewirkt, dass beim Pumpen von Blut in der Blutpumpe das Gleitlager gespült und aus diesem Wärme abtransportiert wird.

Die WO 2016/146661 A1 beschreibt eine Blutpumpe mit einem Laufrad, das mittels eines Drehlagers drehbar innerhalb einem Pumpengehäuse gehalten ist. Durch das Laufrad erstreckt sich ein Auswaschkanal, der ein Auswaschen oder Spülen des Drehlagers ermöglicht.

Die WO 2008/017289 A2 zeigt eine Blutpumpe mit einem Laufrad. Das Laufrad umfasst eingearbeitete Spülkanäle mittels denen Strömungsstagnationen vermieden werden und somit die Thrombengefahr in der Blutpumpe herabgesetzt wird.

Die WO 2017/021465 A1 offenbart eine Blutpumpe. Die Blutpumpe umfasst ein Laufrad, das in einem Pumpengehäuse der Blutpumpe angeordnet ist.

Aufgabe der Erfindung ist es, eine Lagervorrichtung für ein Herzunterstützungssystem bereitzustellen, die ohne komplexe Schaufelstrukturen und/oder Schläuche mit zusätzlichen Spülpumpen für das Spülen mit einem Fluid auskommt, und ein Verfahren zum Spülen einer Lagervorrichtung für ein Herzunterstützungssystem anzugeben, das gewährleistet, dass aus der Lagervorrichtung bei einem Betrieb des Herzunterstützungssystems ausreichend Wärme abgeführt werden kann.

Diese Aufgabe wird durch die in Anspruch 1 angegebene Lagervorrichtung und das in Anspruch 14 angegebene Verfahren gelöst. Vorteilhafte Ausführungsformen der Erfindung sind in den abhängigen Ansprüchen angegeben.

Eine erfindungsgemäße Lagervorrichtung für ein Herzunterstützungssystem enthält eine Standeinheit sowie einen Impeller und weist einen zwischen dem Impeller und der Standeinheit ausgebildeten Zwischenraum zum Führen eines Spülfluidstroms aus einem Fluid auf. Die Standeinheit hat einen in den Impeller ragenden Teilabschnitt, der ausgeformt ist, den Impeller um eine Drehachse drehbar zu lagern. Der Impeller ist ausgebildet, sich bei einem Betrieb des Herzunterstützungssystems zum Fördern eines Pumpenfluidstroms aus dem Fluid in eine Flussrichtung um eine mit der Drehachse fluchtende Längsachse zu drehen, wobei der Impeller wenigstens einen Spülausgang für das Ausleiten des Spülfluidstroms aus dem Zwischenraum aufweist. Der wenigstens eine Spülausgang weist eine Austrittsöffnung für das Austreten des Spülfluidstroms auf, die einen Öffnungsquerschnitt hat, bei dem an wenigstens einer Stelle ein Öffnungsquerschnittsnormalenvektor eine der Drehachse abgewandte und zu der Drehachse radiale Richtungskomponente aufweist. Der Impeller befindet sich in einem Gehäuse mit einem Gehäuseabschnitt, an den in einem Anschlussabschnitt ein Zulaufschlauch für das Zuführen des Fluids angeschlossen ist, wobei der Gehäuseabschnitt zwei durch den Anschlussabschnitt mit dem Gehäuseabschnitt verbindende Stege begrenzte Austrittsöffnungen für das Austreten des Pumpenfluidstroms hat, an die der Anschlussabschnitt angeschlossen ist, und wobei die radiale Richtungskomponente des Öffnungsquerschnittsnormalenvektors in einer zu der Drehachse senkrechten Ebene liegt, welche die Austrittsöffnungen des Gehäuses schneidet.

Der wenigstens eine Spülausgang in dem Impeller kann derart ausgeformt sein, dass ein Rotieren des Impellers um die Drehachse bei dem Betrieb des Herzunterstützungssystems aufgrund einer auf das Fluid in dem wenigstens einen Spülausgang wirkenden Zentrifugalkraft das Austreiben des Fluids aus dem Zwischenraum durch den Spülausgang hindurch zu wenigstens einer Austrittsöffnung bewirkt, wobei der Spülfluidstrom aus dem Zwischenraum ausgeleitet wird.

In dem Impeller können mehrere Spülausgänge ausgeformt sein. Bevorzugt ist der wenigstens eine Spülausgang entlang einer die Längsachse des Impellers schneidende oder hierzu windschief angeordnete Achse erstreckt. Insbesondere kann der wenigstens eine Spülausgang als eine Röhre ausgeformt sein. Die wenigstens eine Austrittsöffnung des Spülausgangs kann z. B. in einem den in den Impeller ragenden Teilabschnitt der Standeinheit umschließenden Mantelabschnitt des Impellers angeordnet sein. Insbesondere kann die wenigstens eine Austrittsöffnung des Spülausgangs in einem Übergangsabschnitt zwischen einem Bereich eines Propellers des Impellers und einem den in den Impeller ragenden Teilabschnitt der Standeinheit umschließenden Mantelabschnitt des Impellers angeordnet sein.

Es ist auch möglich, dass der Impeller eine Mehrzahl von Spülausgängen aufweist, wobei die wenigstens einen Austrittsöffnungen der Spülausgänge zumindest teilweise in einem Übergangsabschnitt zwischen einem Bereich eines Propellers des Impellers und einem den in den Impeller ragenden Teilabschnitt der Standeinheit umschließenden Mantelabschnitt des Impellers angeordnet sind.

Zu bemerken ist, dass eine Anzahl der Spülausgänge in dem Impeller einem Vielfachen der Anzahl der Schaufeln des Impellers entsprechen kann. Zu bemerken ist auch, dass die Lagervorrichtung einen Spüleingang aufweisen kann, der im montierten Zustand der Gleitlagervorrichtung in den Zwischenraum mündet. Der Spüleingang kann dabei z. B. als ein Spalt zwischen einer Basis der Standeinheit und einem den in den Impeller ragenden Teilabschnitt der Standeinheit umschließenden Mantelabschnitt des Impellers ausgeformt sein.

Zu bemerken ist, dass der Spüleingang auch als wenigstens ein eine die Längsachse des Impellers schneidende oder hierzu windschief verlaufende Richtung erstreckter Eingangskanal ausgeformt sein kann. Die Lagervorrichtung kann auch einen Spüleingang mit mehreren Eingangskanälen aufweisen.

Der Spüleingang kann insbesondere bezüglich des Spülausgangs in der Flussrichtung des Pumpenfluidstroms gesehen stromabwärts angeordnet sein.

Der Gehäuseabschnitt kann Stege für das Verbinden mit einem Anschlussabschnitt für das Anschließen eines Zulaufschlauchs aufweisen, wobei die Stege wenigstens eine Austrittsöffnung des Gehäuseabschnitts begrenzen.

Eine erfindungsgemäße Lagervorrichtung kann als eine Gleitlagervorrichtung ausgebildet sein, die für das Lagern einer sich drehenden Komponente ein Gleitlager enthält, oder als eine Magnetlagervorrichtung ausgebildet sein, in der eine sich drehende Komponente magnetisch gelagert ist.

Eine erfindungsgemäße Gleitlagervorrichtung weist eine Standeinheit und einen Impeller auf. Die Standeinheit ist ausgeformt, den Impeller drehbar zu lagern. Der Impeller ist ausgebildet, sich bei einem Betrieb eines Herzunterstützungssystems zum Fördern eines Pumpenfluidstroms zu drehen. Der Impeller ist ausgeformt, zumindest einen Teilabschnitt der Standeinheit im montierten Zustand der Gleitlagervorrichtung zu umschließen. Zwischen dem Teilabschnitt und dem Impeller ist ein Zwischenraum zum Führen eines Spülfluidstroms angeordnet. In dem Impeller ist zumindest ein Spülausgang ausgeformt, um im Betrieb des Herzunterstützungssystems den Spülfluidstrom mittels Zentrifugalkraft aus dem Zwischenraum auszuleiten.

Eine erfindungsgemäße Gleitlagervorrichtung für ein Herzunterstützungssystem ermöglicht insbesondere, dass diese basierend auf einer Ausnutzung der Zentrifugalkraft gespült werden kann. Dazu kann ein Impeller der Gleitlagervorrichtung einen mit dem Impeller rotierenden Spülausgang aufweisen, um die Zentrifugalkraft am rotierenden Spülausgang als treibende Kraft für das Spülen der Gleitlagervorrichtung zu nutzen. Das Spülen der Gleitlagervorrichtung ist bei einem Betrieb des Herzunterstützungssystems vorteilhaft, um Wärme abzuführen und eine Thrombosebildung zu verhindern.

Durch eine Spülung, bei der die Zentrifugalkraft genützt wird und daher die Spülrate im Wesentlichen nur von der Drehzahl des Herzunterstützungssystems und nicht von der statischen Druckdifferenz zwischen Spülungsein- und -ausgang abhängt, ist vorteilhafterweise das Risiko einer Thrombosebildung geringer, weil die Spülrate wesentlich weniger vom Druckverlust im Blutkreislauf beeinflusst wird und damit robuster eingestellt werden kann. Zudem muss keine äußere Druckdifferenz über das Spülsystem aufgeprägt werden.

Zudem ermöglicht die Ausnutzung der Zentrifugalkraft mittels des Spülausgangs in dem Impeller eine kompakte Bauweise der Gleitlagervorrichtung, was insbesondere für die Verwendung der Gleitlagervorrichtung in Verbindung mit dem Herzunterstützungssystem vorteilhaft ist.

Bei dem Herzunterstützungssystem kann es sich beispielsweise um eine Herzpumpe wie ein linksventrikuläres Unterstützungssystem, ein rechtsventrikuläres Unterstützungssystem oder ein biventrikuläres Unterstützungssystem handeln. Unter der Standeinheit kann eine sich nicht drehende Komponente der Gleitlagervorrichtung verstanden werden. Der Impeller kann eine sich drehende Komponente wie ein Laufrad sein. Im montierten Zustand der Gleitlagervorrichtung kann der Impeller zumindest einen Teilabschnitt der Standeinheit umschließen, wodurch die Gleitlagervorrichtung beispielsweise als zylindrisches Gleitlager ausgebildet sein kann. Im implantierten Zustand des Herzunterstützungssystems kann der Impeller im Blut gelagert sein. Der zu fördernde Pumpenfluidstrom kann ein Blutstrom sein, der beispielsweise von dem Herzunterstützungssystem gepumpt und mittels des Herzunterstützungssystems generiert wird. Im montierten Zustand kann zwischen dem Impeller und dem Teilabschnitt der Standeinheit ein Zwischenraum in Form eines Spalts entstehen. Der Spülausgang kann als Bohrung oder eine andere Art von Durchgangsöffnung in dem Impeller realisiert sein. Der Spülausgang kann ausgeformt sein, den Spülfluidstrom von dem Zwischenraum durch einen Abschnitt des Impellers zu leiten, um den Spülfluidstrom aus dem Zwischenraum auszuleiten. Es können auch zwei oder mehr Spülausgänge in dem Impeller ausgeformt sein.

Gemäß einer Ausführungsform kann der Spülausgang bezüglich einer Längsachse des Impellers geneigt sein, die insbesondere einer Drehachse des Impellers entspricht. Dies ist zum Ausnutzen der Zentrifugalkraft von Vorteil, um ein Spülen der Gleitlagervorrichtung zu bewirken. Der Spülausgang kann dabei eine Längserstreckungsachse aufweisen, die bezüglich der Längsachse des Impellers geneigt ist. Die Längserstreckungsachse des Spülausgangs kann auch rechtwinklig bezüglich der Längsachse des Impellers geneigt sein.

Der Spülausgang kann gemäß einer Ausführungsform als eine Röhre mit einer Austrittsöffnung ausgeformt sein. Vorteilhafterweise ist der Spülausgang somit kostensparend beispielsweise als eine Bohrung in dem Impeller realisierbar, was zudem eine kompakte Bauweise der Gleitlagervorrichtung ermöglicht.

Die Austrittsöffnung kann gemäß einer Ausführungsform in einem den Teilabschnitt der Standeinheit umschließenden Mantelabschnitt des Impellers oder in einem Übergangsabschnitt zwischen einem Bereich eines Propellers des Impellers und dem Teilabschnitt angeordnet sein. Der Übergangsabschnitt kann beispielsweise als Verjüngung des Mantelabschnitts in Richtung des Propellers ausgeformt sein. Alternativ kann die Austrittsöffnung auch im Bereich des Propellers angeordnet sein. Mittels der Positionierung der Austrittsöffnung kann das Potential der Zentrifugalkraft eingestellt werden, wodurch vorteilhafterweise die Spülwirkung zum Spülen der Gleitlagervorrichtung eingestellt werden kann.

Zudem kann der Impeller gemäß einer Ausführungsform eine Mehrzahl von Spülausgängen aufweisen. Die Austrittsöffnungen der Spülausgänge können zumindest teilweise in dem Übergangsabschnitt angeordnet sein. Im montierten Zustand der Gleitlagervorrichtung können die Spülausgänge bezüglich der Standeinheit beispielsweise radial auswärts verlaufen. Die Austrittsöffnungen können gleichmäßig beabstandet um den Übergangsabschnitt umlaufend angeordnet sein. Diese Positionierung der Spülausgänge und der Austrittsöffnungen ist in Bezug auf die gleichmäßige Durchspülung des Zwischenraumes und in Bezug auf die Darstellung eines möglichst großen Querschnittes der Spülausgänge vorteilhaft.

In dem Impeller kann gemäß einer Ausführungsform zumindest ein Paar Spülausgänge ausgeformt sein. Die Spülausgänge des zumindest einen Paars können bezüglich einer Längsachse des Impellers einander gegenüberliegend angeordnet sein. Die Ausformung des gegenüberliegenden Paars Spülausgänge ist vorteilhaft, um eine Unwucht des drehenden Propellers vorzubeugen.

Eine Anzahl der Spülausgänge in dem Impeller kann einem Vielfachen der Anzahl der Schaufeln des Impellers entsprechen. Beispielhaft werden die Spülausgänge in Form von Spülbohrungen genauso periodisch angeordnet wie die Beschaufelung des Impellers. Dadurch kann einer Unwucht vorgebeugt werden. In diesem Fall folgt aus beispielsweise zwei Schaufeln ein Vielfaches von zwei als Anzahl der Spülausgänge.

Gemäß einer Ausführungsform kann die Gleitlagervorrichtung auch einen Spüleingang zum Einleiten des Spülfluidstroms aufweisen. Im montierten Zustand der Gleitlagervorrichtung kann der Spüleingang in den Zwischenraum münden. Mittels der wirkenden Zentrifugalkraft kann der Spülfluidstrom den Zwischenraum und somit auch das Lager der Gleitlagervorrichtung spülen, auch ohne dass eine statische Druckdifferenz zwischen dem Spüleingang und dem Spülausgang bereitgestellt wird.

Der Spüleingang kann gemäß einer Ausführungsform auch als ein Spalt zwischen einer Basis der Standeinheit und einem den Teilabschnitt der Standeinheit umschließenden Mantelabschnitt des Impellers ausgeformt sein. Zusätzlich oder alternativ kann der Spüleingang als ein Eingangskanal in dem Impeller ausgeformt sein. Der Eingangskanal kann gegenüber einer Drehachse des Impellers geneigt sein. Ferner kann der Spülausgang durch mehrere Eingangskanäle mit mindestens einem geneigten Eingangskanal in dem Impeller ausgeformt sein. Dadurch kann zumindest eine Seite des Spüleingangs stehend und eine Seite drehend ausgeformt sein. Der Spülfluidstrom kann an der stehenden Seite des Spüleingangs, z. B. an einer Wand der Standeinheit angesaugt werden. Wenn der Spüleingang als ein Eingangskanal in dem Impeller ausgeformt ist, kann der Spüleingang zumindest abschnittsweise in dem rotierenden Körper des Impellers ausgeformt sein. Ein in dem Zwischenraum teileingeschlossener Teil des Spülfluidstroms kann durch den Spüleingang eingeleitet und durch den Spülausgang wieder ausgeleitet werden, um beispielsweise Wärme an der Standeinheit aufzunehmen und abzuführen. Vorteilhafterweise wird der Zentrifugaldruck verstärkt, wenn der Spüleingang nicht oder nur teilweise im rotierenden Körper, dem Impeller, liegt.

Ferner kann der Spüleingang bezüglich des Spülausgangs in Flussrichtung des Pumpenfluidstroms stromabwärts angeordnet sein. Durch das Einleiten des Spülfluidstroms entlang der Standeinheit und entlang des Impellers kann vorteilhafterweise auch bei einem gleichen Druckniveau am Spüleingang und Spülausgang aufgrund der Drehung des Spülfluidstroms am Spülausgang ein konstantes Spülen der Gleitvorrichtung eingestellt werden.

Es wird zudem ein Herzunterstützungssystem mit einer Ausführungsform der vorstehend genannten Gleitlagervorrichtung vorgestellt. Das Herzunterstützungssystem kann beispielsweise eine linksventrikuläre Herzunterstützungspumpe sein. Zudem kann das Herzunterstützungssystem zum minimalinvasiven transfemoralen oder transaortalen Einführen beispielsweise eine längliche, zylindrische Form aufweisen.

Ferner wird ein Verfahren zum Herstellen einer als eine Gleitlagervorrichtung oder als eine Magnetlagervorrichtung ausgebildete Lagervorrichtung für ein Herzunterstützungssystem vorgestellt. Das Verfahren weist folgende Schritte auf:

Bereitstellen einer Standeinheit, die ausgeformt ist, einen Impeller drehbar zu lagern, und des Impellers, der ausgebildet ist, sich bei einem Betrieb des Herzunterstützungssystems zum Fördern eines Pumpenfluidstroms zu drehen;

Ausformen zumindest eines Spülausgangs in dem Impeller, wobei der Spülausgang ausgeformt ist, bei dem Betrieb des Herzunterstützungssystems einen Spülfluidstrom mittels Zentrifugalkraft aus der Lagervorrichtung auszuleiten; und

Montieren des Impellers und der Standeinheit, um die Lagervorrichtung herzustellen, wobei zumindest ein Teilabschnitt der Standeinheit von dem Impeller umschlossen wird, und wobei zwischen dem Teilabschnitt und dem Impeller ein Zwischenraum zum Führen des Spülfluidstroms angeordnet ist.

Durch Ausführen des Verfahrens ist eine Ausführungsform der vorstehend genannten Lagervorrichtung vorteilhaft herstellbar.

Im Folgenden wird die Bedingung dafür dargelegt, dass die Spülung durch Wirkung der Zentrifugalkraft funktioniert:

Die Spülung ist dabei unabhängig von der statischen Druckdifferenz. Zum Spülen der Gleitlagervorrichtung wird die Zentrifugalkraft genutzt, es werden keine externe Pumpe oder zusätzlichen Geometrien oder Strukturen zur Erzeugung einer statischen Druckdifferenz benötigt. Dazu ist es erforderlich, dass die mechanische Energiebilanz aufgrund der kinetischen Rotationsenergie am Austritt, an der Austrittsöffnung des Spülausgangs positiv ist, d. h. die mechanische Energie der Strömung am Austritt muss größer als am Eintritt, am Spüleingang sein. Im Folgenden wird dies anhand von Formeln nach dem Satz von Bernoulli verdeutlicht:

$$\frac{p_{Austritt}}{Dichte} - \frac{v_{Austritt}^2}{2} < \frac{p_{Eintritt}}{Dichte} - \frac{v_{Eintritt}^2}{2}$$

Wenn v gleich der Rotationsgeschwindigkeit ist, und der Spüleingang nicht der Rotation unterliegt folgt:

$$\frac{p_{Austritt}}{Dichte} - \frac{v_{Austritt}^2}{2} < \frac{p_{Eintritt}}{Dichte}$$

umgestellt:

$$\frac{\left(p_{Austritt} - p_{Eintritt}\right)}{Dichte} < \frac{v_{Austritt}^2}{2}$$

mit der Rotationsgeschwindigkeit $v = 2\,\pi\,R\,n$ und mit n gleich der Drehzahl in Umdrehungen/Sekunde folgt

$$\frac{\left(p_{Austritt} - p_{Eintritt}\right)}{Dichte} < 2\,(\pi R n)^2$$

woraus wiederum folgt:

$$\text{statische Druckdifferenz} \ll 2\,(\pi R n)^2 * Dichte$$

Für Wasser entspricht der "Zentrifugaldruck" einem Druckunterschied von ca. 5 bar bei einem Radius von 1 cm und einer Drehzahl von 30000 Umdrehungen/Minute. Der beschriebene Ansatz ist bei diesem Zahlenbeispiel daher wirksam, wenn der statische Druckunterschied ca. nur 500 mbar beträgt ("viel größer" als Faktor zehn interpretiert).

Zum Erreichen des Spülens der Gleitlagervorrichtung mittels Zentrifugalkraft ist ein drehendes System erforderlich, mit Systemgrenzen, dem "Ein-" und "Austritt" in Richtung normal zur Drehachse, nach außen zeigend. Der Spülpfad des Spülfluidstroms verläuft dabei zwischen dem drehenden Körper, dem Mantelabschnitt des Impellers, und dem relativ dazu ruhenden Körper, der Standeinheit. Der Spülfluidstrom bewegt sich dazu gemäß dem hier gezeigten Ausführungsbeispiel entlang des Pfades, als entlang des Zwischenraums zum Spülausgang. Am Austritt des Spülausgangs strömt der Spülfluidstrom aus dem Spülpfad heraus. Zur Aufprägung der Zentrifugalkraft über den gesamten Querschnitt liegt die Austrittberandung des Spülausgangs innerhalb des rotierenden Körpers, innerhalb des Mantelabschnitts. Der Querschnittsnormalenvektor soll eine Komponente in radialer Richtung haben, was beispielsweise an der Stirnseite einer zylindrischen Gleitlagervorrichtung nicht gegeben ist, wohl aber in radialer Richtung, d. h. wenn der Mantelabschnitt angebohrt wird.

Die Erfindung erstreck sich auch auf ein Herzunterstützungssystem, in der es eine vorstehend beschriebene Lagervorrichtung gibt.

Bei einem erfindungsgemäßen Verfahren zum Spülen eines Zwischenraums für das Führen eines Spülfluidstroms mit einem Fluid in einer Lagervorrichtung für ein Herzunterstützungssystem, wobei der Zwischenraum wenigstens einen Spüleingang für das Einleiten des Spülfluidstroms und wenigstens einen Spülausgang für das Ausleiten des Spülfluidstroms aufweist und wobei der Zwischenraum zwischen einem um eine Drehachse drehbaren Impeller zum Fördern eines Pumpenfluidstroms und einer Standeinheit für das drehbare Lagern des Impellers ausgebildet ist, bei dem das Fluid durch den wenigstens einen Spüleingang in den Zwischenraum eingeleitet wird, wird das Fluid mittels einer auf dieses in dem wenigstens einen Spülausgang wirkenden Zentrifugalkraft zu der Drehachse aus dem Zwischenraum durch den Spülausgang hindurch zu wenigstens einer Austrittsöffnung ausgetrieben. Der wenigstens eine Spülausgang weist eine Austrittsöffnung für das Austreten des Spülfluidstroms auf, die einen Öffnungsquerschnitt hat, bei dem an wenigstens einer Stelle ein Öffnungsquerschnittsnormalenvektor eine der Drehachse abgewandte und zu der Drehachse radiale Richtungskomponente aufweist. Der Impeller befindet sich in einem Gehäuse mit einem Gehäuseabschnitt, an den in einem Anschlussabschnitt ein Zulaufschlauch für das Zuführen des Fluids angeschlossen ist, wobei der Gehäuseabschnitt zwei durch Stege begrenzte Austrittsöffnungen für das Austreten des Pumpenfluidstroms hat, an die der Anschlussabschnitt angeschlossen ist. Die Richtung des Spülfluidstroms hat an den Austrittsöffnungen des Spülausgangs eine radiale Richtungskomponente hat, die in einer zu der Drehachse senkrechten Ebene liegt, welche die Austrittsöffnung des Gehäuses schneidet.

Vorteilhafte Ausführungsbeispiele der Erfindung sind nachfolgend anhand von schematischen Zeichnungen näher beschrieben.

Es zeigen:

Fig. 1     eine erste Gleitlagervorrichtung für ein Herzunterstützungssystem mit einem Impeller und mit einer Standeinheit als Schnitt;

Fig. 2     einen Abschnitt eines Herzunterstützungssystems mit der ersten Gleitlagervorrichtung;

Fig. 3     eine Seitenansicht der ersten Gleitlagervorrichtung;

Fig. 4     eine Rückansicht des Impellers in der Richtung des Pfeils IV aus Fig. 3;

Fig. 5     weitere mögliche Bauformen eines Impellers in einer Gleitlagervorrichtung für ein Herzunterstützungssystem;

Fig. 6     einen Zwischenraum mit verschiedenen Spülfluidvolumina in unterschiedlichen Gleitlagervorrichtungen für ein Herzunterstützungssystem bei unterschiedlichen Ausformungen von Spülausgängen;

Fig. 7     eine weitere Gleitlagervorrichtung mit einem Impeller und mit einer Standeinheit;

Fig. 8     die weitere Gleitlagervorrichtung mit einem Impeller und mit einer Standeinheit als Schnitt;

Fig. 9     einen Ausschnitt einer weiteren Gleitlagervorrichtung für ein Herzunterstützungssystem in einer Schnittansicht;

Fig. 10    den Ausschnitt der weiteren Gleitlagervorrichtung für ein Herzunterstützungssystem der Fig. 9 in einer Draufsicht;

Fig. 11    einen Ausschnitt einer weiteren Gleitlagervorrichtung für ein Herzunterstützungssystem in einer Schnittansicht; und

Fig.12    ein Ablaufdiagramm eines Verfahrens zum Herstellen einer Gleitlagervorrichtung.

In der nachfolgenden Beschreibung günstiger Ausführungsbeispiele der vorliegenden Erfindung werden für die in den verschiedenen Figuren dargestellten gleichen oder ähnlich wirkenden Elemente identische Bezugszeichen verwendet, wobei auf eine wiederholte Beschreibung dieser Elemente verzichtet wird.

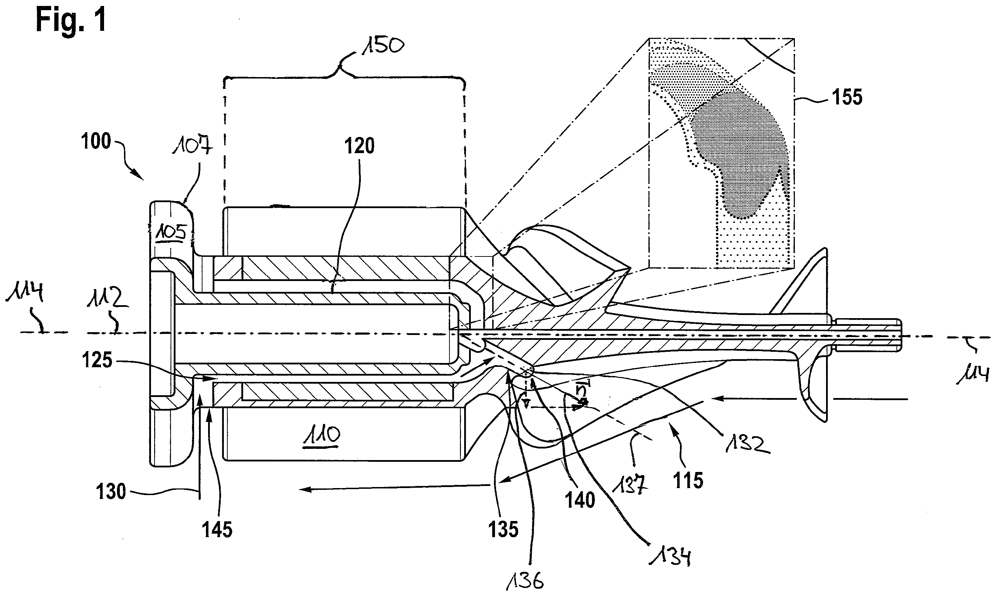

Fig. 1 zeigt eine schematische Darstellung einer als eine Gleitlagervorrichtung ausgebildete Lagervorrichtung 100 für ein Herzunterstützungssystem gemäß einem Ausführungsbeispiel. Die Lagervorrichtung 100 weist eine Standeinheit 105 und einen Impeller 110 auf. Die Standeinheit 105 ist ausgeformt, den Impeller 110 um eine Drehachse 112 drehbar zu lagern, die koaxial zu der Längsachse 114 des Impellers 110 verläuft. Der Impeller 110 ist ausgebildet, sich bei einem Betrieb des Herzunterstützungssystems zum Fördern eines Pumpenfluidstroms 115 um die Drehachse 112 zu drehen. Im hier gezeigten montierten Zustand der Gleitlagervorrichtung umschließt der Impeller 110 zumindest einen Teilabschnitt 120 der Standeinheit 105. Zwischen dem Teilabschnitt 120 und dem Impeller 110 ist ein Zwischenraum 125 zum Führen eines Spülfluidstroms 130 angeordnet. In dem Impeller 110 ist zumindest ein Spülausgang 135 ausgeformt. Der Spülausgang 135 ist ausgeformt, bei dem Betrieb des Herzunterstützungssystems den Spülfluidstrom 130 mittels Zentrifugalkraft aus dem Zwischenraum 125 auszuleiten.

Der Spülausgang 135 hat eine Ausrittsöffnung 140 für das Austreten des Spülfluidstroms 130, die einen Öffnungsquerschnitt 132 aufweist, bei dem an wenigstens einer Stelle ein Öffnungsquerschnittsnormalenvektor 134 eine der Drehachse 112 abgewandte und zu der Drehachse 112 radiale Richtungskomponente 136 aufweist.

Gemäß dem hier gezeigten Ausführungsbeispiel ist der Spülausgang 135 bezüglich der zu der Drehachse 112 koaxialen Längsachse 114 des Impellers 110 geneigt. Der Spülausgang 135 weist dabei eine Achse 137 auf, entlang welcher der Spülausgang 135 erstreckt ist und die damit eine Längserstreckungsachse des Spülausgangs 135 ist, die bezüglich der Längsachse 114 des Impellers 110 geneigt ist und mit dieser einen spitzen Winkel α einschließt. Zu bemerken ist, dass diese Achse 137 zu der Längsachse 114 des Impellers 110 grundsätzlich auch windschief verlaufen kann.

Zudem ist der Spülausgang 135 gemäß dem hier gezeigten Ausführungsbeispiel als Röhre mit einer Austrittsöffnung 140 ausgeformt. Die Austrittsöffnung 140 ist an einem von dem Zwischenraum 125 abgewandten Ende der Röhre angeordnet.

Auch weist die Gleitlagervorrichtung 100 gemäß dem hier gezeigten Ausführungsbeispiel einen Spüleingang 145 zum Einleiten des Spülfluidstroms 130 auf. Der Spüleingang 145 mündet in dem hier gezeigten montierten Zustand der Lagervorrichtung 100 in den Zwischenraum 125.

Der Spüleingang 145 ist gemäß dem hier gezeigten Ausführungsbeispiel als ein Spalt zwischen einer Basis 107 der Standeinheit 105 und einem den Teilabschnitt 120 der Standeinheit 105 umschließenden Mantelabschnitt 150 des Impellers 110 ausgeformt. Zu bemerken ist, dass der Spüleingang grundsätzlich auch als ein Eingangskanal in dem Impeller 110 ausgeformt sein kann.

Bei der in der Fig. 1 gezeigten Gleitlagervorrichtung ist der Spüleingang 145 wie in dem hier gezeigten Ausführungsbeispiel bezüglich des Spülausgangs 135 in der Flussrichtung des Pumpenfluidstroms 115 stromabwärts angeordnet. Die Fig. 1 zeigt einen Spülfluidstrom 130 mit einem Spülungspfad zum Spülen der Lagervorrichtung 100, der von dem Spüleingang 145 durch den Zwischenraum 125 zu dem Spülausgang 135 mit der Austrittsöffnung 140 verläuft.

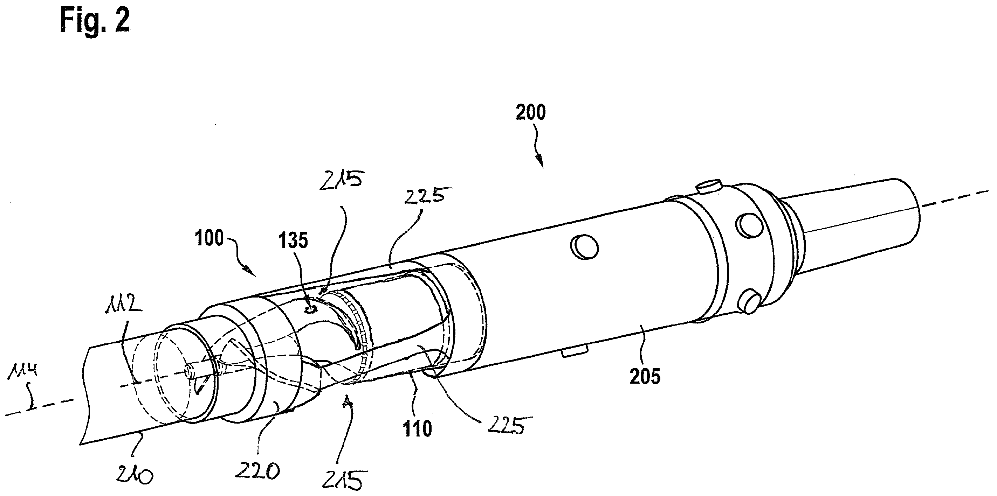

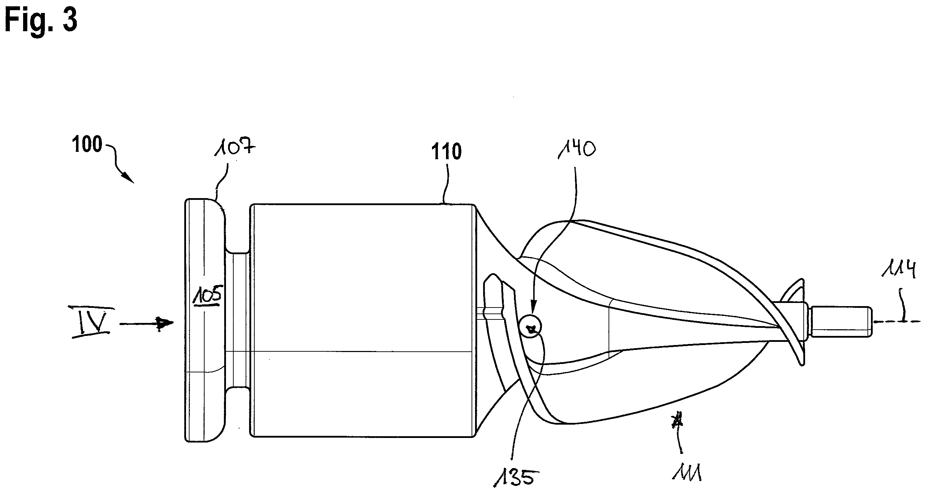

Die Fig. 2 zeigt in einer perspektivischen Ansicht einen Abschnitt eines Herzunterstützungssystems 200 mit der Gleitlagervorrichtung 100 in Form einer Linksventrikulären Herzunterstützungspumpe (LVAD-Herzpumpe). Die Fig. 3 ist eine Seitenansicht der Lagervorrichtung 100.

Im Folgenden wird die Lagervorrichtung 100 und deren Funktion in einem Herzunterstützungssystem näher beschrieben:
Der Impeller 110 ist ein Laufrad, das in der Lagervorrichtung 100 des Herzunterstützungssystems 200 eine drehende Komponente bildet, die per Gleitlager oder magnetisch gelagert ist, wobei die drehende Komponente für die Wärmeabfuhr oder zur Reibreduktion über einem Fluid gelagert ist. Wenn der Impeller 110 bei einem Betrieb des Herzunterstützungssystems direkt im Blut gelagert ist, wie dies beispielsweise bei der in der Fig. 2 gezeigten Linksventrikulären Herzunterstützungspumpe (LVAD-Herzpumpe) im implantierten Zustand des Herzunterstützungssystems der Fall ist, so ist es vorteilhaft, die Lagervorrichtung 100 zu spülen, um eine Wärmeabfuhr zu erreichen und eine Thrombosebildung ("Blutverklumpung") zu verhindern. Zum Ermöglichen des robusten Spülens der Gleitlagervorrichtung 100 ist eine konstante Durchströmung notwendig. Das Spülen der Gleitlagervorrichtung 100 verhindert eine Thrombosebildung. Dazu kann eine Pumpenkonstruktion (wie z. B. Leitbleche) verwendet werden, die mechanische Energie in hydrodynamische umwandelt. Die in der Fig. 1 und Fig. 2 gezeigte Gleitlagervorrichtung 100 ermöglicht, lediglich mit einer Bohrung in Form des Spülausgangs 135 die Zentrifugalkraft am mit dem Impeller 110 rotierenden Spülausgang 135 auszunutzen, wobei die Zentrifugalkraft die treibende Kraft für die Spülung darstellt. Eine solche Struktur ist kostengünstig in der Fertigung.

Alternativ sind auch eine Mehrzahl an Spülausgängen 135 an unterschiedlichen Stellen des Impellers 110 zum Ausnutzen der Zentrifugalkraft realisierbar, wie anhand nachfolgender Figuren gezeigt.

Mittels eines Ausführungsbeispiels der hier gezeigten Lagervorrichtung 100 ist ein Einleiten in Form einer Absaugung des Spülfluidstroms 130 aufgrund der Zentrifugalkraft am Spülausgang 135 realisierbar. Dies geschieht konstruktiv dadurch, dass der Spülausgang 135 so ausgeformt ist, dass der Spülausgang 135 von der drehenden Komponente, dem Impeller 110, umschlossen ist, z. B. durch eine Bohrung als Spülausgang 135, während die Eingangsseite in Form des Spüleingangs 145 nicht oder nur teilweise, z. B. nur einseitig, der Drehung unterliegt. Dies wird durch ein Ausformen des Spüleingangs 145 mit zumindest einem Abschnitt der Standeinheit 105 als Wandabschnitt erreicht. In diesem Fall hat die statistische Druckdifferenz praktisch keinen Einfluss auf den Spülfluss des Spülfluidstroms 130, weshalb die Spülwirkung der Lagervorrichtung 100 im Wesentlichen von der Zentrifugalkraft und der Drehzahl der Pumpe des Herzunterstützungssystems bestimmt ist. Die Spülwirkung der Lagervorrichtung 100 ist damit weitgehend unabhängig von weiteren potentiellen Einflussgrößen wie dem Betrag des Massenstroms oder der Höhe des Druckaufbaus durch oder über das Herzunterstützungssystem. Zum Spülen der Lagervorrichtung 100 ist damit kein statischer Druckunterschied nötig. Daher ist ein Positionieren des Spülausgangs 135 in dem hier beispielhaft trompetenförmig ausgeformten Impeller 110 mit stark unterschiedlichen Durchmessern in Bezug auf eine Längserstreckungsachse 114 des Impellers 110 unterschiedlich realisierbar, wobei ein Positionieren des Spülausgangs 135 weit stromaufwärts der Längserstreckung des Impellers 110 unterbleiben kann. Zum Bewirken des Spülens der Gleitlagervorrichtung 100 sind zudem keine komplexen Strukturen wie z. B. ein Pumpenrad oder die Beaufschlagung einer Druckdifferenz in der oder um die Gleitlagervorrichtung 100 notwendig. Aufgrund der Unabhängigkeit vom Pumpenfluss, der hier gezeigten Pumpenströmung des Pumpenfluidstroms 115, ist das Spülen der Lagervorrichtung 100 ohne Spülungsausfall möglich, solange die Drehung des Impellers 110 erfolgt.

In dem hier beschriebenen Ausführungsbeispiel weist die Lagervorrichtung 100 als ein drehendes Teil den Impeller 110 auf, der mit der Standeinheit 105 als stehendem Teil ein zylindrisches Gleitlager bildet. Die Spülwirkung der Lagervorrichtung 100 basiert auf der Zentrifugalkraft aufgrund einer Drehung am Spülausgang 135. Voraussetzung dafür ist, dass am Spüleingang 145 wie hier gezeigt mindestens eine Seite steht, hier die innere Seite in Gestalt der Standeinheit 105. Dadurch ist selbst bei einem vergleichbaren oder gleichen Druckniveau am Spüleingang 145 und am Spülausgang 135 aufgrund der Drehung beider Seiten des in dem rotierenden Impeller 110 ausgeformten Spülausgangs 135 bzw. des Fluidvolumens des Spülausgangs 135 eine konstante Spülung der Gleitlagervorrichtung 100 einstellbar. Zudem ist es mittels des hier gezeigten Ausführungsbeispiels der Lagervorrichtung 100 möglich, ein teileingeschlossenes Volumen, das hier in dem Block 155 gezeigt ist, das beispielhaft um das Festlager der Standeinheit 105 angeordnet ist, mittels der Kombination einer drehenden und einer stehenden Seite zu spülen. Dies geschieht, da an der drehenden Seite des Impellers aufgrund der molekularen Haftbedingung der Spülfluidstrom 130 beschleunigt wird. Der Spülfluidstrom 130 wird entlang der Wand des Zwischenraums 125 aufgrund der Zentrifugalkraft hin zu einem größeren Durchmesser beschleunigt, wodurch der Spülfluidstrom 130 an der stehenden Seite des Zwischenraums 125 in Form einer Wand der Standeinheit 105 angesaugt wird. Dies führt dazu, dass das teileingeschlossene Fluid des Spülfluidstroms 130 gespült wird und so z. B. Wärme am Festlager der Standeinheit 105 aufgenommen und abgeführt werden kann.

Das in der Fig. 2 gezeigte Herzunterstützungssystem 200 umfasst einen Gehäuseabschnitt 205. In dem Gehäuseabschnitt 205 des Herzunterstützungssystems 200 befindet sich der Impeller 110 der Lagervorrichtung 100. In dem Herzunterstützungssystem 200 ist der Impeller 110 in einem Gehäuseabschnitt 205 angeordnet, an den ein Zulaufschlauch 210 für das Zuführen des Fluids angeordnet ist. In dem Gehäuseabschnitt 205 des Gehäuses des Herzunterstützungssystems gibt es Austrittsöffnungen 215 für das Austreten des Pumpenfluidstroms 115. Für das Anschließen des Zulaufschlauchs 210 gibt es ein dem Herzunterstützungssystem 200 einen Anschlussabschnitt 220, der an Stege 225 des Gehäuseabschnitts 205 angeschlossen ist, welche zwei Austrittsöffnungen 215 für das Austreten von durch ein Rotieren des Impellers 110 in dem Herzunterstützungssystem 200 geförderten Fluid aus dem Gehäuseabschnitt 205 begrenzen.

Der Gehäuseabschnitt 205 des Herzunterstützungssystems 200 weist einen zylinderförmigen, länglichen Aufbau mit im Wesentlichen konstantem Außendurchmesser zur einfachen Platzierung mittels eines Katheters in einem Blutgefäß, etwa der Aorta, auf. Die hier gezeigte längliche axiale Bauart ermöglicht eine transfemorale Implantation des Herzunterstützungssystems 200. Entsprechend ist die Gleitlagervorrichtung 100 in einer Fensteröffnung in dem Gehäuseabschnitt 205 so angeordnet, dass die drehende Laufrad-Komponente, der Impeller 110, im implantierten Zustand des Herzunterstützungssystems 200 im Blut gelagert ist. Durch die axiale Bauart des Herzunterstützungssystems 200 wird der Impeller 110 in Bezug auf die Längsachse 114 des Impellers 110, die einer Längsachse des Herzunterstützungssystems 200 entspricht, axial angeströmt. Der Spülausgang 135 in dem Impeller 110 ist dabei in dem Bereich 111 eines Propellers des Impellers 110 angeordnet, wobei der Spülausgang 135 durch ein Bohrloch bzw. eine Durchgangsbohrung oder andere Art von Durchgangsloch im Impeller 110 realisiert ist.

Die Fig. 3 zeigt die Gleitlagervorrichtung 100 mit der Standeinheit 105 und dem Impeller 110 im montierten Zustand, wobei die Standeinheit 105 das nichtdrehende Gegenstück zu dem sich drehenden Impeller 110 bildet. Die Standeinheit 105 hat einen in Richtung des Impellers 110 weisenden Abschnitt mit einer Verjüngung. Der verjüngte Abschnitt der Standeinheit 105 ist zum überwiegenden Teil von dem Impeller 110 umschlossen. Die Standeinheit 105 ist mit dem Impeller 110 verbunden und lagert den Impeller 110 drehbar. In dem Impeller 105 ist der Spülausgang 135 ausgeformt, der eine Austrittsöffnung 140 aufweist. Die Austrittsöffnung 140 des Spülausgangs ist hier beispielhaft im Bereich des Propellers des Impellers 110 angeordnet.

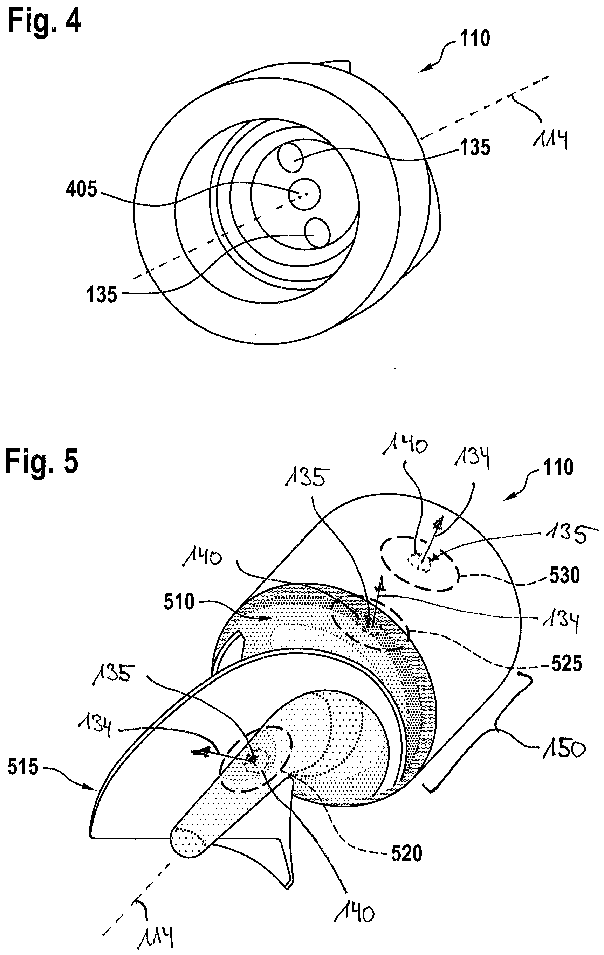

Die Fig. 4 zeigt eine perspektivische Rückansicht des Impellers in der Richtung des Pfeils IV aus Fig. 3. Als Rückseite des Impellers 110 ist die dem Propeller des Impellers 110 abgewandte Seite des Impellers 110 gezeigt, die mit der Standeinheit 105 der Lagervorrichtung koppelbar ist. Zum Verbinden des Impellers 110 mit der Standeinheit 105 weist der Impeller 110 hier ein Kugellager 405 zur Lagerung des Impellers 110 auf. Ferner sind beispielhaft die hier als Austrittsbohrungen gestalteten Spülausgänge 135 des Impellers 110 zu sehen, die mit dem in der Fig. 1 gezeigten Zwischenraum 125 kommunizieren.

Gemäß dem hier gezeigten Ausführungsbeispiel ist in dem Impeller 110 zumindest ein Paar Spülausgänge 135 ausgeformt. Die Spülausgänge 135 des zumindest einen Paars sind bezüglich einer Längsachse 114 des Impellers 110 einander gegenüberliegend angeordnet. Beispielhaft sind die Spülausgänge 135 des Paars in Bezug auf die Drehachse 112 des Impellers 110 gleichmäßig beabstandet, d. h. sie haben einen zu der Drehachse 112 koaxialen Längsachse 114 des Impellers 110 symmetrischen Verlauf.

Die Fig. 5 zeigt weitere mögliche Bauformen eines Impellers 110 in einer Lagervorrichtung für ein Herzunterstützungssystem, die als eine Gleitlagervorrichtung oder als eine Magnetlagervorrichtung ausgebildet sein kann. Es ist eine perspektivische Ansicht des Impellers 110 gezeigt, wobei unterschiedliche, beispielhafte Positionierungen einer Austrittsöffnung 140 des Spülausgangs 135 in dem Impeller 110 sowie ein jeweiliger Öffnungsquerschnittsnormalenvektor 134 und die Längsachse 114 des Impellers 110 kenntlich gemacht sind.

Gemäß einem Ausführungsbeispiel ist die Austrittsöffnung 140 des Spülausgangs 135 in einem den Teilabschnitt der Standeinheit umschließenden Mantelabschnitt 150 des Impellers 110 angeordnet. Alternativ ist die Austrittsöffnung des Spülausgangs in einem Übergangsabschnitt 510 zwischen einem Bereich eines Propellers 515 des Impellers 110 und dem Mantelabschnitt 505 angeordnet.

In der vorliegenden Figur ist eine Potentialabschätzung für das Ausführungsbeispiel, wo ist die größte Saugkraft und damit eine geeignete Stelle für die Positionierung des Spülausganges und der Austrittsöffnung des Spülausgangs vorliegt, gezeigt. Es sind beispielhaft drei Bereiche 520, 525 und 530 zur Anordnung der Austrittsöffnung des Spülausgangs in dem Impeller 110 gezeigt. Der Bereich 520 ist im Bereich des Propellers 515 angeordnet. Der Bereich 525 markiert beispielhaft eine Position der Austrittsöffnung des Spülausgangs 135 in dem Übergangsabschnitt 510. Der Bereich 530 markiert eine beispielhafte Positionierung der Austrittsöffnung des Spülausgangs im Mantelabschnitt 150. Gemäß der hier gezeigten Potentialabschätzung wird beim Positionieren des Spülausgangs 135 und der Austrittsöffnung 140 im Bereich 530 eine vorteilhafte Spülwirkung in einer Lagervorrichtung mit einem solchen Impeller 110 und einer Standeinheit 105 erzielt, da zwischen dem Spüleingang und dem Spülausgang die Zentrifugalkraft ausreicht, um die Spülung zu treiben.

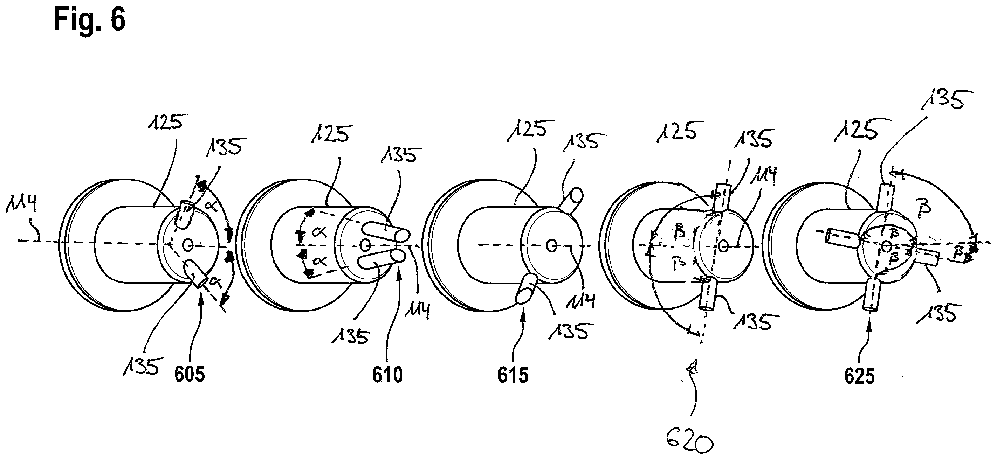

Die Fig. 6 zeigt den Zwischenraum 125 mit verschiedenen Spülfluidvolumina in unterschiedlichen, als Gleitlagervorrichtung oder als Magnetlagervorrichtung ausgebildeten Lagervorrichtungen für ein Herzunterstützungssystem bei unterschiedlichen Ausformungen von Spülausgängen, wobei die Spülausgänge 135 unterschiedlich ausgeformt sind. Der Spülausgang 135, durch den der Spülfluidstrom verläuft, hat hier unterschiedlichen Ausformungen 605, 610, 615, 620, 625. In dem Impeller dieser Gleitlagervorrichtungen ist dabei zumindest ein Paar Spülausgänge 135 ausgeformt, wobei die Spülausgänge 135 des zumindest einen Paars in einer Lagervorrichtung bezüglich der mit der Drehachse 112 fluchtenden Längsachse 114 des Impellers 110 einander gegenüberliegend angeordnet sind. Die hier gezeigten Ausformungen 605, 610, 615, 620, 625 der Spülausgänge zeigen beispielhaft jeweils das Paar Spülausgänge. In einer ersten Ausformung 605 verlaufen die Spülausgänge des Paars bezüglich der Längsachse 114 des Impellers in einem stumpfen Winkel $\alpha$ geneigt, von der Längsachse 114 des Impellers radial ausgehend, wobei ein Ansatzpunkt der Spülausgänge 135 an die Längsachse 114 angenähert ausgeformt ist. In einer zweiten Ausformung 610 verlaufen die Spülausgänge 135 des Paars bezüglich der Längsachse 114 des Impellers in einem spitzen Winkel $\alpha$ geneigt, die Spülausgänge 135 des Paars sind entsprechend einander zugeneigt ausgeformt. Eine dritte Ausformung 615 entspricht der ersten Ausformung 605 mit Ausnahme des Ansatzpunktes der Spülausgänge 135, die weiter voneinander entfernt angeordnet sind als die Ansatzpunkte der Spülausgänge der ersten Ausformung 605. In einer vierten Ausformung 620 verlaufen die Spülausgänge 135 des Paars in einem rechten Winkel $\beta$ zu der Längsachse 114 des Impellers. Eine fünfte Ausformung 625 zeigt beispielhaft zwei Paar Spülausgänge 135 die bezüglich der Längsachse 114 des Impellers einander gegenüberliegend angeordnet und gleichmäßig voneinander beabstandet angeordnet sind. Die zwei Paar Spülausgänge 135 verlaufen wie das in der vierten Ausformung 620 gezeigte Paar in einem rechten Winkel $\beta$ zu der Längsachse 114 des Impellers.

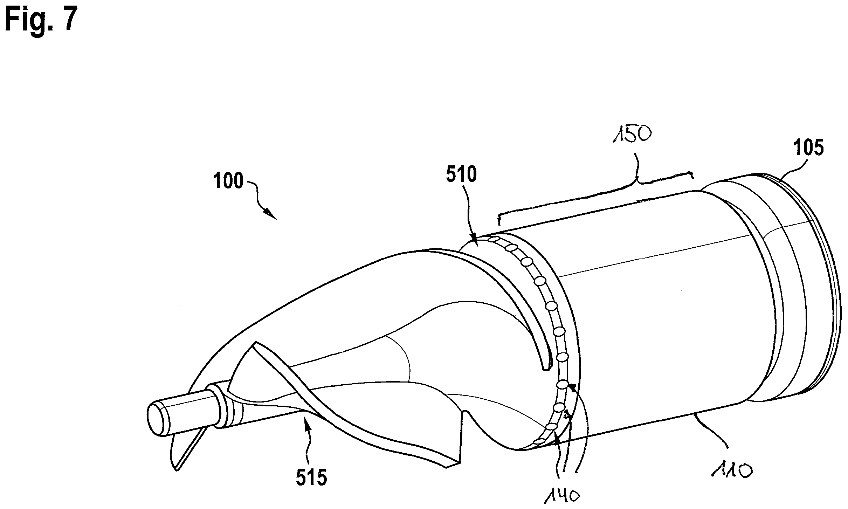

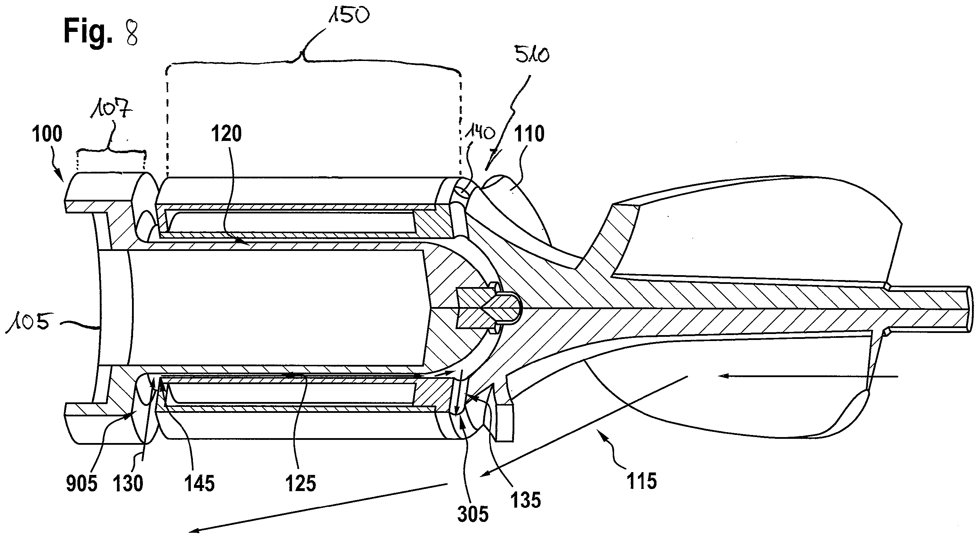

Fig. 7 zeigt eine weitere Gleitlagervorrichtung 100 für ein Herzunterstützungssystem. Es ist eine perspektivische Ansicht der Gleitlagervorrichtung 100 im montierten Zustand gezeigt, in dem der Impeller die Standeinheit 105 abschnittsweise umschließt. In der Fig. 8 ist diese Gleitlagervorrichtung 100 als Schnitt gezeigt. Die hier gezeigte Gleitlagervorrichtung 100 ähnelt der anhand der vorhergehenden Figuren beschriebenen Gleitlagervorrichtung. Gemäß dem hier gezeigten Ausführungsbeispiel weist der Impeller 110 eine Mehrzahl von Spülausgängen 135 auf. Die Austrittsöffnungen 140 der Spülausgänge sind zumindest teilweise in dem Übergangsabschnitt 510 zwischen dem Propeller 515 und dem Mantelabschnitt 505 angeordnet. Beispielhaft sind die Austrittsöffnungen 140 gleichmäßig beabstandet, um dem Übergangsabschnitt 510 umlaufend angeordnet. Fig. 7 zeigt eine Ausnutzung der Spülungsposition der Mehrzahl an Spülausgängen mit der als am größten ermittelten Saugkraft.

Fig. 8 zeigt eine weitere Gleitlagervorrichtung 100 für ein Herzunterstützungssystem. Es ist eine Schnittdarstellung einer Seitenansicht der Gleitlagervorrichtung 100 gezeigt. Die Standeinheit 105 wird teilweise von dem Mantelabschnitt 150 des Impellers 110 umschlossen. Im Übergangsbereich bzw. Übergangsabschnitt 510 zwischen dem Propeller des Impellers 110 und dem Mantelabschnitt 150 ist die Mehrzahl von Austrittsöffnungen 140 von Spülausgängen 135 angeordnet. Es ist die Strömungsrichtung des Pumpenfluidstroms 115 gezeigt sowie der Strömungspfad des Spülfluidstroms 130. Der Spülfluidstrom 130 wird durch den Spüleingang 145 eingeleitet, der gemäß dem hier gezeigten Ausführungsbeispiel als ein Spalt 905 zwischen der Basis 107 der Standeinheit 105 und dem den Teilabschnitt 120 der Standeinheit 105 umschließenden Mantelabschnitt 505 des Impellers 110 ausgeformt ist. Der Spülfluidstrom 130 wird dann mittels Zentrifugalkraft durch den Zwischenraum 125 zu einer der Austrittsöffnungen 140 der Mehrzahl an Spülausgängen 135 geleitet, um die Gleitlagervorrichtung 100 zu spülen.

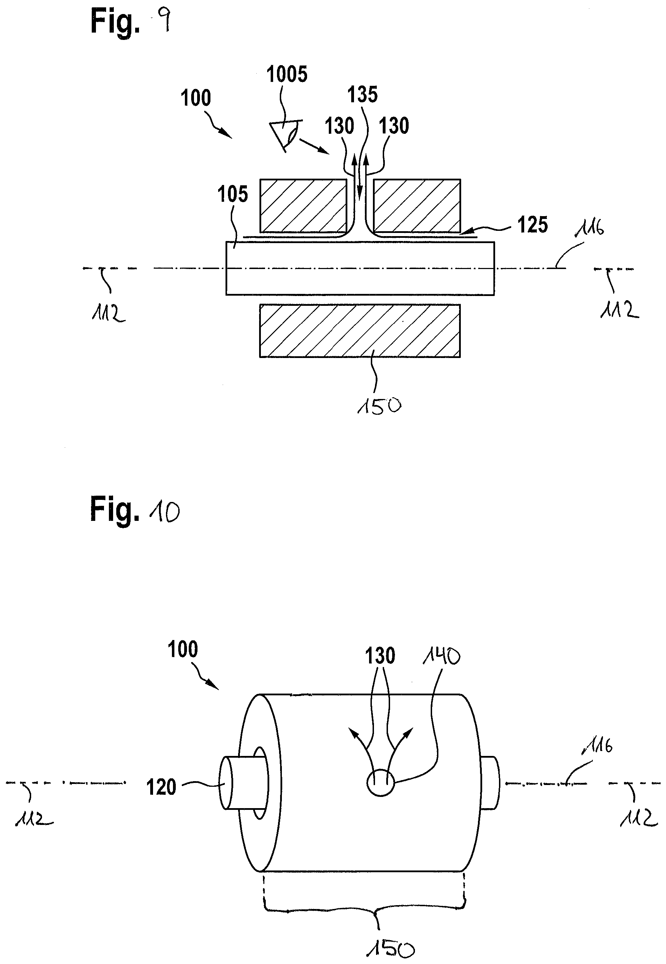

Fig. 9 zeigt eine schematische Darstellung eines Ausschnitts einer Gleitlagervorrichtung 100 für ein Herzunterstützungssystem gemäß einem Ausführungsbeispiel. Gezeigt ist ein Querschnitt eines Teils der Gleitlagervorrichtung 100 mit dem vom Mantelabschnitt 150 des Impellers umschlossenen Teilabschnitt der Standeinheit 105. Die Einformung des Spülausgangs 135 soll hier zeigen, dass die Spülausgänge auch spiegelunsymmetrisch angeordnet sein können.

Es ist ein Teil des Spülungspfads des Spülfluidstroms 130 gezeigt, der durch den Zwischenraum 125 zu dem Spülausgang 135 strömt, und aus der Austrittsöffnung des Spülausgangs 135 ausgeleitet wird. Das Ausströmen des Spülfluidstroms ist in der nachfolgenden Fig. 10 anhand einer Draufsicht aus der hier mit dem Pfeil 1005 markierten Richtung gezeigt.

Fig. 10 zeigt eine schematische Darstellung eines Ausschnitts einer Gleitlagervorrichtung 100 für ein Herzunterstützungssystem gemäß einem Ausführungsbeispiel. Es ist eine Draufsicht auf den in der vorhergehenden Fig. 9 markierten Ausschnitt der Gleitlagervorrichtung 100 gezeigt. Der Spülausgang 135 ist in dem Mantelabschnitt 150 radial zu einer mit der Drehachse 112 in der Lagervorrichtung fluchtenden Längserstreckungsachse 116 des von dem Mantelabschnitt 505 umschlossenen Teilabschnitts der Standeinheit 105 angeordnet. Der Spülfluidstrom 130 tritt an der Austrittsöffnung 140 des Spülausgangs aus dem Mantelabschnitt 150 aus.

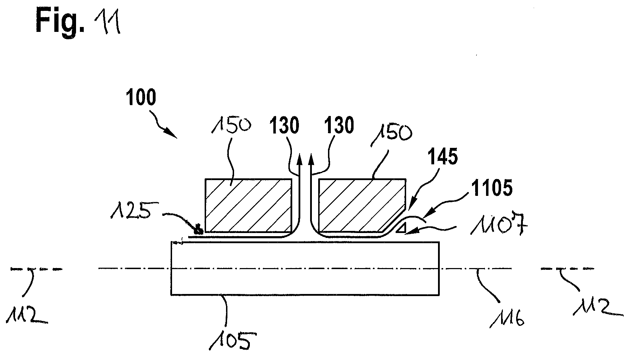

Fig. 11 zeigt eine schematische Darstellung eines Ausschnitts einer Gleitlagervorrichtung 100 für ein Herzunterstützungssystem gemäß einem Ausführungsbeispiel. Gemäß dem hier gezeigten Ausführungsbeispiel ist der Spüleingang 145 in den Zwischenraum 125 durch mehrere Eingangskanäle realisiert, nämlich durch den Kanal 1105 und den Kanal 1107. Dies soll zudem demonstrieren, dass die Eingangsrichtung nicht nur zwangsläufig in der mit der Richtung der mir der Drehachse 112 der Lagervorrichtung 100 fluchtenden Längserstreckungsachse 116 der Lagervorrichtung 100 orientiert sein muss sondern auch dazu geneigt erfolgen kann. Wenn der Spüleingang 145 so gestaltet ist, dass dort keine Zentrifugalkraft wirkt, in dem beispielweise die Berandung des Spüleingangs 145 nicht im oder nur teilweise im rotierenden Körper liegt, wie in dem hier gezeigten Ausführungsbeispiel des Spüleingangs 145 als Eingangskanal 1105, der teilweise im Mantelabschnitt 150 ausgeformt ist, dann verstärkt sich vorteilhafterweise der Zentrifugaldruck.

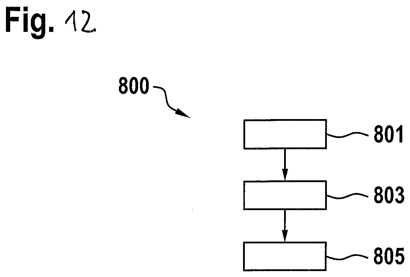

Fig. 12 zeigt ein Ablaufdiagramm eines Verfahrens 800 zum Herstellen einer als eine Gleitlagervorrichtung oder als eine Magnetlagervorrichtung ausgebildete Lagervorrichtung für ein Herzunterstützungssystem gemäß einem Ausführungsbeispiel. Das Verfahren 800 weist einen Schritt 801 des Bereitstellens, einen Schritt 803 des Ausformens und einen Schritt 805 des Montierens auf. Im Schritt 801 des Bereitstellens wird eine Standeinheit bereitgestellt, die ausgeformt ist, einen Impeller drehbar zu lagern. Zudem wird im Schritt 801 der Impeller bereitgestellt, der ausgebildet ist, sich bei einem Betrieb des Herzunterstützungssystems zum Fördern eines Pumpenfluidstroms zu drehen. Im

Schritt 803 des Ausformens wird in dem Impeller zumindest ein Spülausgang ausgeformt, der ausgeformt ist, bei dem Betrieb des Herzunterstützungssystems einen Spülfluidstrom mittels Zentrifugalkraft aus der Gleitlagervorrichtung auszuleiten. Im Schritt 805 des Montierens werden der Impeller und die Standeinheit montiert, um die Gleitlagervorrichtung herzustellen. Dabei wird zumindest ein Teilabschnitt der Standeinheit von dem Impeller umschlossen. Zudem ist zwischen dem Teilabschnitt und dem Impeller ein Zwischenraum zum Führen des Spülfluidstroms angeordnet. Bei dem Betrieb des Herzunterstützungssystems wird der Spülfluidstrom mittels Zentrifugalkraft aus dem Zwischenraum in den Spülausgang geleitet, und von dort aus der Lagervorrichtung ausgeleitet, um die Lagervorrichtung zu spülen.

Zusammenfassend ist insbesondere folgendes festzuhalten: Die Erfindung betrifft eine Lagervorrichtung 100 für ein Herzunterstützungssystem. Die Lagervorrichtung 100 umfasst eine Standeinheit 105 und einen Impeller 110. Die Standeinheit 105 ist ausgeformt, den Impeller 110 drehbar zu lagern. Der Impeller 110 ist ausgebildet, sich bei einem Betrieb des Herzunterstützungssystems zum Fördern eines Pumpenfluidstroms 115 zu drehen. Der Impeller 110 ist ausgeformt, zumindest einen Teilabschnitt 120 der Standeinheit 105 im montierten Zustand der Lagervorrichtung 100 zu umschließen, wobei zwischen dem Teilabschnitt 120 und dem Impeller 110 ein Zwischenraum 125 zum Führen eines Spülfluidstroms 130 angeordnet ist. In dem Impeller 110 ist zumindest ein Spülausgang 135 ausgeformt. Der Spülausgang 135 ist ausgeformt, bei dem Betrieb des Herzunterstützungssystems den Spülfluidstrom 130 mittels Zentrifugalkraft aus dem Zwischenraum 125 auszuleiten.

**Bezugszeichenliste**

| | |
|---|---|
| 100 | Gleitlagervorrichtung |
| 105 | Standeinheit |
| 107 | Basis |
| 110 | Impeller |
| 111 | Bereich eines Propellers des Impellers |
| 112 | Drehachse |
| 114 | Längsachse |
| 115 | Pumpenfluidstrom |
| 116 | Längserstreckungsachse |
| 120 | Teilabschnitt der Standeinheit |
| 125 | Zwischenraum |
| 130 | Spülfluidstrom |
| 132 | Öffnungsquerschnitt |
| 134 | Öffnungsquerschnittsnormalenvektor |
| 135 | Spülausgang |
| 136 | Richtungskomponente |
| 137 | Achse |
| 140 | Austrittsöffnung |
| 145 | Spüleingang |
| 150 | Mantelabschnitt |
| 155 | Block |
| 200 | Herzunterstützungssystem |
| 205 | Gehäuseabschnitt |
| 210 | Zulaufschlauch |
| 215 | Austrittsöffnung |
| 220 | Anschlussabschnitt |
| 225 | Steg |
| 405 | Kugellager |
| 505 | Mantelabschnitt |
| 510 | Übergangsabschnitt |
| 515 | Propeller |
| 520, 525, 530 | Bereich |
| 605, 610, 615, 620, 625 | Ausformung |
| 800 | Verfahren |
| 801 | Schritt des Bereitstellens |
| 803 | Schritt des Ausformens |
| 805 | Schritt des Montierens |
| 905 | Spalt |
| 1005 | Pfeil |
| 1105, 1107 | Eingangskanal |

**Patentansprüche**

1. Lagervorrichtung (100) für ein Herzunterstützungssystem (200) mit einer Standeinheit (105), mit einem Impeller (110) und mit einem zwischen dem Impeller (110) und der Standeinheit (105) ausgebildeten Zwischenraum (125) zum Führen eines Spülfluidstroms (130) aus einem Fluid,

wobei die Standeinheit (105) einen in den Impeller (110) ragenden Teilabschnitt (120) aufweist und ausgeformt ist, den Impeller (110) um eine Drehachse (112) drehbar zu lagern;

wobei der Impeller (110) ausgebildet ist, sich bei einem Betrieb des Herzunterstützungssystems (200) zum Fördern eines Pumpenfluidstroms (115) aus dem Fluid in eine Flussrichtung um eine mit der Drehachse (112) fluchtende Längsachse (114) zu drehen, und

wobei der Impeller (110) wenigstens einen Spülausgang (135) für das Ausleiten des Spülfluidstroms (130) aus dem Zwischenraum (125) aufweist,

wobei der wenigstens eine Spülausgang (135) eine Austrittsöffnung (140) für das Austreten des Spülfluidstroms (130) aufweist, die einen Öffnungsquerschnitt (132) hat, bei dem an wenigstens einer Stelle ein Öffnungsquerschnittsnormalenvektor (134) eine der Drehachse (112) abgewandte und zu der Drehachse (112) radiale Richtungskomponente (136) aufweist,

und wobei sich der Impeller (110) in einem Gehäuse mit einem Gehäuseabschnitt (205) befindet, an den in einem Anschlussabschnitt (220) ein Zulaufschlauch (210) für das Zuführen des Fluids angeschlossen ist,

**dadurch gekennzeichnet, dass** der Gehäuseabschnitt (205) zwei durch den Anschlussabschnitt (220) mit dem Gehäuseabschnitt (205) verbindende Stege (225) begrenzte Austrittsöffnungen (215) für das Austreten des Pumpenfluidstroms (115) hat, an die der Anschlussabschnitt (220) angeschlossen ist,

wobei die radiale Richtungskomponente (136) des Öffnungsquerschnittsnormalenvektors (134) in einer zu der Drehachse (112) senkrechten Ebene liegt, welche die Austrittsöffnungen (215) des Gehäuses schneidet.

2. Lagervorrichtung (100) gemäß Anspruch 1, **dadurch gekennzeichnet, dass** in dem Impeller (110) mehrere Spülausgänge (135) ausgeformt sind, und/oder dass der wenigstens eine Spülausgang (135) entlang einer die Längsachse (114) des Impellers (110) schneidende oder hierzu windschief angeordnete Achse (137) erstreckt ist, und/oder dass der wenigstens eine Spülausgang (135) als eine Röhre ausgeformt ist.

3. Lagervorrichtung (100) gemäß einem der Ansprüche 1 oder 2, **dadurch gekennzeichnet, dass** die wenigstens eine Austrittsöffnung (140) des Spülausgangs (135) in einem den in den Impeller (110) ragenden Teilabschnitt (120) der Standeinheit (105) umschließenden Mantelabschnitt (150) des Impellers (110) angeordnet ist.

4. Lagervorrichtung (100) gemäß einem der Ansprüche 1 bis 3, **dadurch gekennzeichnet, dass** die wenigstens eine Austrittsöffnung (140) des Spülausgangs (135) in einem Übergangsabschnitt (510) zwischen einem Bereich eines Propellers (515) des Impellers (110) und einem den in den Impeller (110) ragenden Teilabschnitt (120) der Standeinheit (105) umschließenden Mantelabschnitt (150) des Impellers (110) angeordnet ist.

5. Lagervorrichtung (100) gemäß einem der Ansprüche 1 bis 3, **dadurch gekennzeichnet, dass** der Impeller (110) eine Mehrzahl von Spülausgängen (135) aufweist, wobei die wenigstens einen Austrittsöffnungen (140) der Spülausgänge (135) zumindest teilweise in einem Übergangsabschnitt (510) zwischen einem Bereich eines Propellers (515) des Impellers (110) und einem den in den Impeller (110) ragenden Teilabschnitt (120) der Standeinheit (105) umschließenden Mantelabschnitt (150) des Impellers (110) angeordnet sind.

6. Lagervorrichtung (100) gemäß einem der vorangegangenen Ansprüche, **dadurch gekennzeichnet, dass** eine Anzahl der Spülausgänge (135) in dem Impeller (110) einem Vielfachen der Anzahl der Schaufeln des Impellers (110) entspricht.

7. Lagervorrichtung (100) gemäß einem der vorangegangenen Ansprüche, **gekennzeichnet durch** einen Spüleingang (145), der im montierten Zustand der Lagervorrichtung (100) in den Zwischenraum (125) mündet.

8. Lagervorrichtung (100) gemäß einem der Ansprüche 1 bis 7, **dadurch gekennzeichnet, dass** der Spüleingang (145) als ein Spalt (905) zwischen einer Basis (107) der Standeinheit (105) und einem den in den Impeller (110) ragenden Teilabschnitt (120) der Standeinheit (105) umschließenden Mantelabschnitt (150) des Impellers (110) ausgeformt ist.

9. Lagervorrichtung (100) nach einem der Ansprüche 1 bis 8, **dadurch gekennzeichnet, dass** der Spüleingang (145) als wenigstens ein in eine die Längsachse (114) des Impellers (110) schneidende oder hierzu windschief verlaufende Richtung erstreckter Eingangskanal (1105) ausgeformt ist.

10. Lagervorrichtung (100) nach Anspruch 8 oder 9, **gekennzeichnet durch einen** mehrere Eingangskanäle aufweisenden Spüleingang (145).

**11.** Lagervorrichtung (100) gemäß einem der Ansprüche 7 bis 10, **dadurch gekennzeichnet, dass** der Spüleingang (145) bezüglich des Spülausgangs (135) in der Flussrichtung des Pumpenfluidstroms (115) gesehen stromabwärts angeordnet ist.

**12.** Lagervorrichtung (100) nach einem der Ansprüche 1 bis 11, **dadurch gekennzeichnet, dass** diese als eine Gleitlagervorrichtung, die für das Lagern einer sich drehenden Komponente in Form des Impellers (110) ein Gleitlager enthält, oder als eine Magnetlagervorrichtung ausgebildet ist, in der eine sich drehende Komponente in Form des Impellers (110) magnetisch gelagert ist.

**13.** Herzunterstützungssystem (200) mit einer Lagervorrichtung (100) gemäß einem der vorangegangenen Ansprüche.

**14.** Verfahren zum Spülen eines Zwischenraums (125) für das Führen eines Spülfluidstroms (130) mit einem Fluid in einer Lagervorrichtung (100) für ein Herzunterstützungssystem (200), wobei der Zwischenraum (125) wenigstens einen Spüleingang (145) für das Einleiten des Spülfluidstroms (130) und wenigstens einen Spülausgang (135) für das Ausleiten des Spülfluidstroms (130) aufweist und wobei der Zwischenraum (125) zwischen einem um eine Drehachse (112) drehbaren Impeller (110) zum Fördern eines Pumpenfluidstroms (115) und einer Standeinheit (105) für das drehbare Lagern des Impellers (110) ausgebildet ist, bei dem das Fluid durch den wenigstens einen Spüleingang (145) in den Zwischenraum (125) eingeleitet wird, wobei

das Fluid mittels einer auf dieses in dem wenigstens einen Spülausgang (135) wirkenden Zentrifugalkraft zu der Drehachse (112) aus dem Zwischenraum (125) durch den Spülausgang (135) hindurch zu wenigstens einer Austrittsöffnung (140) ausgetrieben wird,
wobei der wenigstens eine Spülausgang (135) eine Austrittsöffnung (140) für das Austreten des Spülfluidstroms (130) aufweist, die einen Öffnungsquerschnitt (132) hat, bei dem an wenigstens einer Stelle ein Öffnungsquerschnittsnormalenvektor (134) eine der Drehachse (112) abgewandte und zu der Drehachse (112) radiale Richtungskomponente (136) aufweist,
und wobei sich der Impeller (110) in einem Gehäuse mit einem Gehäuseabschnitt (205) befindet, an den in einem Anschlussabschnitt (220) ein Zulaufschlauch (210) für das Zuführen des Fluids angeschlossen ist, **dadurch gekennzeichnet, dass** der Gehäuseabschnitt (205) zwei durch den Anschlussabschnitt (220) mit dem Gehäuseabschnitt (205) verbindende Stege begrenzte Austrittsöffnungen (215) für das Austreten des Pumpenfluidstroms (115) hat, an die der Anschlussabschnitt (220) angeschlossen ist,
wobei die Richtung des Spülfluidstroms (139) an der Austrittsöffnung (140) des Spülausgangs (135) eine radiale Richtungskomponente hat, die in einer zu der Drehachse (112) senkrechten Ebene liegt, welche die Austrittsöffnung (215) des Gehäuses schneidet.

## Claims

**1.** Bearing device (100) for a heart support system (200) comprising a support unit (105), an impeller (110) and an intermediate space (125) formed between the impeller (110) and the support unit (105) for guiding a rinse fluid stream (130) from a fluid,

the support unit (105) having a partial portion (120) projecting into the impeller (110) and being shaped to support the impeller (110) rotatably about a rotation axis (112);
the impeller (110) being designed to rotate about a longitudinal axis (114) aligned with the rotation axis (112) during operation of the heart support system (200) in order to convey a pump fluid stream (115) from the fluid in a flow direction, and
the impeller (110) having at least one rinse outlet (135) for discharging the rinse fluid stream (130) from the intermediate space (125),
the at least one rinse outlet (135) having an outlet opening (140) for the outlet of the rinse fluid stream (130), which has an opening cross-section (132), in which, at least at one point, an opening cross-section normal vector (134) has a directional component (136) facing away from the rotation axis (112) and radial to the rotation axis (112), and the impeller (110) being located in a housing having a housing portion (205) to which an inlet hose (210) for supplying the fluid is connected in a connection portion (220),
**characterized in that** the housing portion (205) has two outlet openings (215) for the outlet of the pump fluid stream (115), which openings are delimited by ribs (225) connecting the connecting portion (220) to the housing portion (205), to which openings the connecting portion (220) is connected,

the radial directional component (136) of the opening cross-section normal vector (134) lying in a plane perpendicular to the rotation axis (112) which plane intersects the outlet openings (215) of the housing.

2. Bearing device (100) according to claim 1, **characterized in that** in the impeller (110) a plurality of rinse outlets (135) are formed, **and/or in that** the at least one rinse outlet (135) extends along an axis (137) intersecting the longitudinal axis (114) of the impeller (110) or arranged at an angle thereto, **and/or in that** the at least one rinse outlet (135) is formed as a tube.

3. Bearing device (100) according to either claim 1 or claim 2,
   **characterized in that** the at least one outlet opening (140) of the rinse outlet (135) is arranged in a casing portion (150) of the impeller (110) enclosing the partial portion (120) of the support unit (105) projecting into the impeller (110).

4. Bearing device (100) according to any of claims 1 to 3,
   **characterized in that** the at least one outlet opening (140) of the rinse outlet (135) is arranged in a transition portion (510) between a region of a propeller (515) of the impeller (110) and a casing portion (150) of the impeller (110) enclosing the partial portion (120) of the support unit (105) projecting into the impeller (110).

5. Bearing device (100) according to any of claims 1 to 3,
   **characterized in that** the impeller (110) has a plurality of rinse outlets (135), the at least one outlet opening (140) of the rinse outlets (135) being arranged at least partially in a transition portion (510) between a region of a propeller (515) of the impeller (110) and a casing portion (150) of the impeller (110) enclosing the partial portion (120) of the support unit (105) projecting into the impeller (110).

6. Bearing device (100) according to any of the preceding claims,
   **characterized in that** a number of rinse outlets (135) in the impeller (110) corresponds to a multiple of the number of blades of the impeller (110).

7. Bearing device (100) according to any of the preceding claims,
   **characterized by** a rinse inlet (145) which opens into the intermediate space (125) when the bearing device (100) is in the mounted state.

8. Bearing device (100) according to any of claims 1 to 7,
   **characterized in that** the rinse inlet (145) is formed as a gap (905) between a base (107) of the support unit (105) and a casing portion (150) of the impeller (110) enclosing the partial portion (120) of the support unit (105) projecting into the impeller (110).

9. Bearing device (100) according to any of claims 1 to 8,
   **characterized in that** the rinse inlet (145) is formed as at least one inlet channel (1105) extending in a direction intersecting the longitudinal axis (114) of the impeller (110) or running at an angle thereto.

10. Bearing device (100) according to either claim 8 or claim 9,
    **characterized by** a rinse inlet (145) having a plurality of inlet channels.

11. Bearing device (100) according to any of claims 7 to 10,
    **characterized in that** the rinse inlet (145) is arranged downstream of the rinse outlet (135) in the flow direction of the pump fluid stream (115).

12. Bearing device (100) according to any of claims 1 to 11,
    **characterized in that** this is designed as a plain bearing device, which contains a plain bearing for supporting a rotating component in the form of the impeller (110), or as a magnetic bearing device, in which a rotating component in the form of the impeller (110) is magnetically mounted.

13. Heart support system (200) comprising a bearing device (100) according to any of the preceding claims.

14. Method for rinsing an intermediate space (125) for guiding a rinse fluid stream (130) with a fluid in a bearing device (100) for a heart support system (200), the intermediate space (125) having at least one rinse inlet (145) for introducing the rinse fluid stream (130) and at least one rinse outlet (135) for discharging the rinse fluid stream (130), and the intermediate space (125) being formed between an impeller (110) rotatable about a rotation axis (112) for conveying a pump fluid stream (115) and a support unit (105) for rotatably supporting the impeller (110), in which the fluid is introduced into the intermediate space (125) through the at least one rinse inlet (145),

the fluid being expelled from the intermediate space (125) through the rinse outlet (135) to at least one outlet opening (140) by means of a centrifugal force acting thereon in the at least one rinse outlet (135) relative to the rotation axis (112),

the at least one rinse outlet (135) having an outlet opening (140) for the outlet of the rinse fluid stream (130), which has an opening cross-section (132), in which, at least at one point, an opening cross-section normal vector (134) has a directional component (136) facing away from the rotation axis (112) and radial to the rotation axis (112), and the impeller (110) being located in a housing having a housing portion (205) to which an inlet hose (210) for supplying the fluid is connected in a connection portion (220),

**characterized in that** the housing portion (205) has two outlet openings (215) for the outlet of the pump fluid stream (115), which openings are delimited by ribs connecting the connecting portion (220) to the housing portion (205), to which openings the connecting portion (220) is connected,

the direction of the rinse fluid stream (139) at the outlet opening (140) of the rinse outlet (135) having a radial directional component which lies in a plane perpendicular to the rotation axis (112), which plane intersects the outlet opening (215) of the housing.

## Revendications

1. Dispositif de palier (100) pour un système d'assistance cardiaque (200) comportant une unité de support (105), comportant un impulseur (110) et un espace intermédiaire (125) conçu entre l'impulseur (110) et l'unité de support (105) pour le guidage d'un flux de fluide de rinçage (130) à partir d'un fluide,

dans lequel l'unité de support (105) présente une section partielle (120) faisant saillie dans l'impulseur (110) et est formée pour supporter l'impulseur (110) de manière rotative autour d'un axe de rotation (112) ;

dans lequel l'impulseur (110) est conçu pour tourner autour d'un axe longitudinal (114) aligné avec l'axe de rotation (112) lors d'un fonctionnement du système d'assistance cardiaque (200) pour le transport d'un flux de fluide de pompe (115), à partir du fluide, dans une direction d'écoulement, et

dans lequel l'impulseur (110) présente au moins une sortie de rinçage (135) pour l'évacuation du flux de fluide de rinçage (130) de l'espace intermédiaire (125),

dans lequel l'au moins une sortie de rinçage (135) présente une ouverture de sortie (140) pour la sortie du flux de fluide de rinçage (130), laquelle ouverture de sortie possède une section transversale d'ouverture (132) dans laquelle, en au moins un endroit, un vecteur normal de section transversale d'ouverture (134) présente une composante directionnelle (136) opposée à l'axe de rotation (112) et radiale par rapport à l'axe de rotation (112), et dans lequel l'impulseur (110) se trouve dans un boîtier comportant une section de boîtier (205) à laquelle est raccordé, dans une section de raccordement (220), un tuyau d'arrivée (210) pour l'amenée du fluide,

**caractérisé en ce que** la section de boîtier (205) possède deux ouvertures de sortie (215) pour la sortie du flux de fluide de pompe (115), délimitées par des nervures (225) reliant la section de raccordement (220) à la section de boîtier (205), la section de raccordement (220) étant raccordée aux ouvertures de sortie,

dans lequel la composante directionnelle (136) radiale du vecteur normal de section transversale d'ouverture (134) est située dans un plan perpendiculaire à l'axe de rotation (112) et coupant les ouvertures de sortie (215) du boîtier.

2. Dispositif de palier (100) selon la revendication 1, **caractérisé en ce que** plusieurs sorties de rinçage (135) sont formées dans l'impulseur (110),

**et/ou en ce que** l'au moins une sortie de rinçage (135) s'étend le long d'un axe (137) coupant l'axe longitudinal (114) de l'impulseur (110) ou disposé en oblique par rapport à celle-ci, **et/ou en ce que** l'au moins une sortie de rinçage (135) est formée comme un tube.

3. Dispositif de palier (100) selon l'une des revendications 1 ou 2,
**caractérisé en ce que** l'au moins une ouverture de sortie (140) de la sortie de rinçage (135) est disposée dans une section d'enveloppe (150) de l'impulseur (110), laquelle section d'enveloppe entoure la section partielle (120) de l'unité de support (105), la section partielle faisant saillie dans l'impulseur (110).

4. Dispositif de palier (100) selon l'une des revendications 1 à 3,
**caractérisé en ce que** l'au moins une ouverture de sortie (140) de la sortie de rinçage (135) est disposée dans une section de transition (510) entre une zone d'une hélice (515) de l'impulseur (110) et une section d'enveloppe (150) de l'impulseur (110), laquelle section d'enveloppe entoure la section partielle (120) de l'unité de support (105), la section partielle faisant saillie dans l'impulseur (110).

5. Dispositif de palier (100) selon l'une des revendications 1 à 3,
**caractérisé en ce que** l'impulseur (110) présente une pluralité de sorties de rinçage (135), dans lequel l'au moins ouverture de sortie (140) des sorties de rinçage (135) est disposée au moins partiellement dans une section de transition (510) entre une zone d'une hélice (515) de l'impulseur (110) et une section d'enveloppe (150) de l'impulseur (110), laquelle section d'enveloppe entoure la section partielle (120) de l'unité de support (105), la section partielle faisant saillie dans l'impulseur (110).

6. Dispositif de palier (100) selon l'une des revendications précédentes,
**caractérisé en ce qu'**un nombre de sorties de rinçage (135) dans l'impulseur (110) correspond à un multiple du nombre d'aubes de l'impulseur (110).

7. Dispositif de palier (100) selon l'une des revendications précédentes,
**caractérisé par** une entrée de rinçage (145) débouchant dans l'espace intermédiaire (125) lorsque le dispositif de palier (100) est monté.

8. Dispositif de palier (100) selon l'une des revendications 1 à 7,
**caractérisé ce que** l'entrée de rinçage (145) est formée comme un interstice (905) entre une base (107) de l'unité de support (105) et une section d'enveloppe (150) de l'impulseur (110), laquelle section d'enveloppe entoure la section partielle (120) de l'unité de support (105), la section partielle faisant saillie dans l'impulseur (110).

9. Dispositif de palier (100) selon l'une des revendications 1 à 8,
**caractérisé en ce que** l'entrée de rinçage (145) est formée comme au moins un canal d'entrée (1105) s'étendant dans une direction coupant l'axe longitudinal (114) de l'impulseur (110) ou s'étendant en oblique par rapport à celui-ci.

10. Dispositif de palier (100) selon la revendication 8 ou 9, **caractérisé par** une entrée de rinçage (145) présentant plusieurs canaux d'entrée.

11. Dispositif de palier (100) selon l'une des revendications 7 à 10,
**caractérisé en ce que** l'entrée de rinçage (145) est disposée en aval par rapport à la sortie de rinçage (135), vu dans la direction d'écoulement du flux de fluide de pompe (115).

12. Dispositif de palier (100) selon l'une des revendications 1 à 11,
**caractérisé en ce qu'**il est conçu sous la forme d'un dispositif de palier lisse qui contient un palier lisse pour le support d'un composant rotatif sous la forme de l'impulseur (110), ou sous la forme d'un dispositif de palier magnétique dans lequel un composant rotatif sous la forme de l'impulseur (110) est supporté magnétiquement.

13. Système d'assistance cardiaque (200) comportant un dispositif de palier (100) selon l'une des revendications précédentes.

14. Procédé permettant de rincer un espace intermédiaire (125) destiné au guidage d'un flux de fluide de rinçage (130) avec un fluide dans un dispositif de palier (100) pour un système d'assistance cardiaque (200), dans lequel l'espace intermédiaire (125) présente au moins une entrée de rinçage (145) pour l'introduction du flux de fluide de rinçage (130) et au moins une sortie de rinçage (135) pour l'évacuation du flux de fluide de rinçage (130), et dans lequel l'espace intermédiaire (125) est conçu entre un impulseur (110) pouvant tourner autour d'un axe de rotation (112) pour le transport d'un flux de fluide de pompe (115) et une unité de support (105) pour le support rotatif de l'impulseur (110), dans lequel le fluide est introduit dans l'espace intermédiaire (125) par l'au moins une entrée de rinçage (145), dans lequel

le fluide est expulsé de l'espace intermédiaire (125) à travers la sortie de rinçage (135) vers au moins une ouverture de sortie (140) par le biais d'une force centrifuge agissant sur le fluide, par rapport à l'axe de rotation (112), dans l'au moins une sortie de rinçage (135),
dans lequel l'au moins une sortie de rinçage (135) présente une ouverture de sortie (140) pour la sortie du flux de fluide de rinçage (130), laquelle ouverture de sortie possède une section transversale d'ouverture (132) dans laquelle, en au moins un endroit, un vecteur normal de section transversale d'ouverture (134) présente une composante directionnelle (136) opposée à l'axe de rotation (112) et radiale par rapport à l'axe de rotation (112), et dans lequel l'impulseur (110) se trouve dans un boîtier comportant une section de boîtier (205) à laquelle est raccordé, dans une section de raccordement (220), un tuyau d'arrivée (210) pour l'amenée du fluide, **caractérisé en ce que** la section de boîtier (205) possède deux ouvertures de sortie (215) pour la sortie du flux de fluide de pompe (115), délimitées par des nervures reliant la section de raccordement (220) à la section de boîtier (205), la section de raccordement (220) étant raccordée aux ouvertures de sortie,

dans lequel la direction du flux de fluide de rinçage (139) au niveau de l'ouverture de sortie (140) de la sortie de rinçage (135) possède une composante de direction radiale qui est située dans un plan perpendiculaire à l'axe de rotation (112), lequel plan coupe l'ouverture de sortie (215) du boîtier.

# Fig. 1

EP 3 833 410 B1

**Fig. 2**

EP 3 833 410 B1

**Fig. 3**

# Fig. 4

# Fig. 5

Fig. 6

Fig. 7

EP 3 833 410 B1

Fig. 8

## Fig. 9

## Fig. 10

**Fig. 11**

# Fig. 12

**EP 3 833 410 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3127562 A1
- WO 2016146661 A1
- WO 2008017289 A2
- WO 2017021465 A1